# FASCICULE DE BREVET EUROPEEN

(11) **EP 1 937 318 B1**
(45) Date de publication et mention de la délivrance du brevet: **22.08.2012**
(21) Numéro de dépôt: 05824598.6
(22) Date de dépôt: 05.12.2005
(51) Int. Cl.: A61L 2/10, A61L 2/26

(54) **ENCEINTE DE DESINFECTION D'INSTRUMENTS MEDICAUX AU MOYEN DE RAYONNEMENT**
DESINFEKTIONSKAMMER FÜR MEDIZINISCHE INSTRUMENTE DURCH STRAHLUNG
MEDICAL INSTRUMENT DISINFECTING CHAMBER VIA RADIATION

(30) Priorité: 21.09.2005 FR 0509649
(43) Date de publication de la demande: 02.07.2008
(73) Titulaire: Germitec, 75008 Paris (FR)
(72) Inventeur: DESHAYS, Clément, F-07120 Ruoms (FR)
(74) Mandataire: Jacobson, Claude
(86) Numéro de dépôt international: PCT/FR2005/003031
(87) Numéro de publication internationale: WO 2007/034040

(56) Documents cités:
- EP-A- 0 839 537
- DE-A1- 3 209 701
- GB-A- 2 364 622
- US-A- 4 772 795
- US-A- 5 185 532
- US-A- 6 039 928
- US-A1- 2002 162 972

## Description

La présente invention concerne une enceinte de désinfection d'instruments médicaux.

Plus particulièrement, l'invention se rapporte à une telle enceinte qui comporte des moyens de suspension d'au moins un instrument dans cette enceinte.

On connaît déjà dans l'état de la technique par exemple par le document EP-A-0 839 537, une enceinte de décontamination pour instruments médicaux délimitée par un fond, au moins une paroi latérale et un couvercle supérieur, chaque instrument comportant une partie active et une partie de raccordement sous la forme d'un câble.

Cette enceinte comporte également une potence s'étendant à l'intérieur et en partie supérieure de l'enceinte, parallèlement au fond et en surplomb du fond, cette potence comportant une pluralité d'organes de suspension, chacun d'eux étant destiné à coopérer avec une partie du câble voisine de la partie active de l'instrument.

De tels moyens de suspension permettent alors de maintenir dans une position sensiblement verticale et sans contact, un instrument dans l'enceinte afin d'en assurer une parfaite désinfection.

On sait en effet que la suspension, sans contact, des instruments lors de leur désinfection assure la qualité de la désinfection. Cependant, ce type de structure présente un certain nombre d'inconvénients notamment au niveau de l'homogénéité de la désinfection en particulier lorsque l'enceinte est équipée de moyens de génération de rayonnement UV de type C par exemple permettant d'assurer cette désinfection des instruments.

Par ailleurs, il peut également arriver pour une raison ou pour une autre que l'instrument qui peut par exemple se présenter sous la forme d'une sonde ou autre, soit entortillé sur lui-même ou déformé de sorte que tout ou partie de l'instrument peut alors se retrouver dans une position et/ou un état défavorables pour sa désinfection.

Le but de l'invention est donc de résoudre ces problèmes.

A cet effet, l'invention a pour objet une enceinte de désinfection d'instruments médicaux, du type comportant des moyens de suspension d'au moins un instrument dans l'enceinte et des moyens de génération dans l'enceinte, d'un rayonnement de désinfection, caractérisée en ce qu'elle comporte des moyens, transparents au rayonnement, de maintien de l'instrument dans un état allongé et une orientation sensiblement verticale, sous les moyens de suspension.

Les moyens de maintien comprennent des moyens de guidage dans l'enceinte.

Ces moyens de guidage comprennent un ou plusieurs organes en forme de peigne de l'instrument répartis sur la hauteur de l'enceinte.

Suivant des modes particuliers de réalisation, l'enceinte selon l'invention peut comporter l'une ou plusieurs des caractéristiques suivantes :
- les moyens de maintien sont adaptés pour maintenir l'instrument sensiblement au centre de l'enceinte ;
- Les moyens de guidage comprennent un ou plusieurs tubes de réception de l'instrument ;
- les moyens de maintien comprennent un poids adapté pour être accroché à l'extrémité libre de l'instrument; et
- les moyens de maintien sont réalisés en oxyde de silicium.

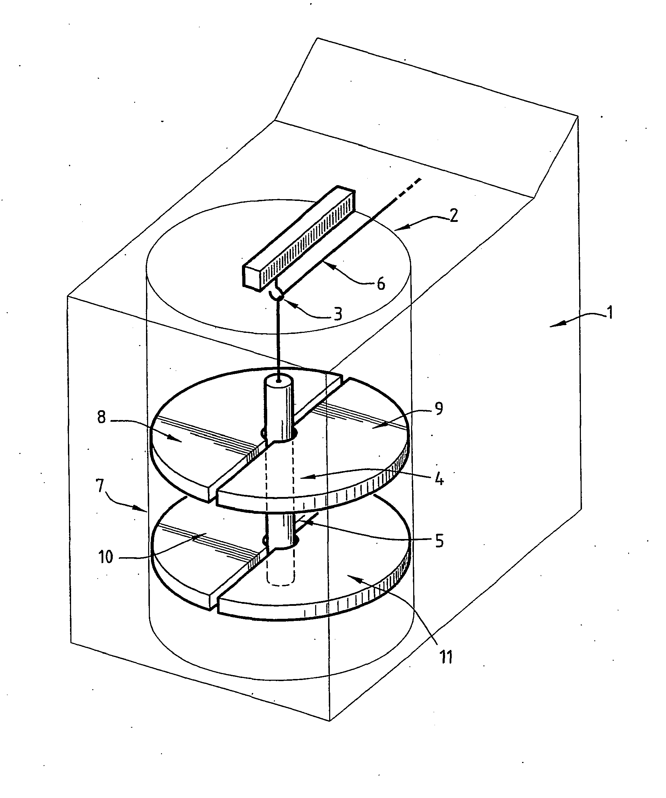

L'invention sera mieux comprise à l'aide de la description qui va suivre donnée uniquement à titre d'exemple et faite en se référant au dessin annexé qui représente un schéma synoptique illustrant la structure et le fonctionnement d'une enceinte de désinfection selon l'invention.

On a en effet illustré sur cette figure, une enceinte de désinfection d'instruments médicaux.

Cette enceinte est désignée par la référence générale 1 sur cette figure et comporte des moyens de génération d'un rayonnement de désinfection, comme par exemple un rayonnement UV de type C par exemple.

Un exemple de réalisation d'une telle enceinte est décrit dans le document EP-A-0839537, auquel on pourra se référer pour les détails de réalisation de celle-ci.

D'autres enceintes de désinfection sont connues de US-A-5,185,532, US-A-2002/0162972 et GB-A-2364622.

L'enceinte décrite dans ce document comporte en fait une potence qui est désignée par la référence générale 2 sur cette figure et qui s'étend à l'intérieur et en partie supérieure de l'enceinte, parallèlement au fond et en surplomb du fond de cette enceinte.

En fait, cette potence entre dans la constitution de moyens de suspension d'au moins un instrument à désinfecter dans l'enceinte.

De manière avantageuse, ces moyens de suspension peuvent par exemple être réglables en hauteur dans l'enceinte par tout mécanisme de réglage connu dans l'état de la technique comme par exemple un mécanisme à vis-écrou manoeuvrable grâce à une molette par un opérateur.

Bien entendu, d'autres modes de réalisation peuvent être envisagés.

Dans l'exemple de réalisation illustré sur la figure, les moyens de suspension comprennent également un organe en forme de crochet désigné par la référence générale 3 auquel peut être suspendu l'instrument désigné par la référence générale 4.

En fait, cet instrument est un instrument médical qui peut se présenter sous la forme d'une sonde ou autre, qui comporte alors par exemple une partie active désignée par la référence générale 5 et un câble de raccordement désigné par la référence générale 6 permettant de suspendre cet instrument.

Lorsque les moyens de génération du rayonnement de désinfection sont activés, ils permettent alors d'assurer la désinfection de l'instrument par action du rayonnement.

La demanderesse a déjà proposé dans d'autres demandes de brevets différents perfectionnements à ce type d'enceinte en prévoyant notamment des formes particulières de l'enceinte, des revêtements particuliers de celle-ci, des déplacements particuliers de l'instrument mais également des moyens permettant d'assurer la traçabilité de l'opération de désinfection.

Pour améliorer encore la qualité de la désinfection dans l'enceinte selon l'invention, il est prévu des moyens, transparents au rayonnement, de maintien de l'instrument dans un état allongé et une orientation sensiblement verticale, sous les moyens de suspension.

Ceci permet par exemple d'éviter toute déformation ou entortillement de l'instrument qui pourrait se traduire par une disposition et/ou un état de celui-ci défavorables à sa désinfection.

Différents modes de réalisation de ces moyens de maintien de l'instrument peuvent être envisagés.

En fait, ces moyens de maintien sont adaptés pour maintenir de préférence l'instrument sensiblement au centre de l'enceinte comme cela est illustré.

Ces moyens de maintien peuvent alors comporter par exemple des moyens de guidage de l'instrument comme ceux illustrés sur cette figure et désignés par la référence générale 7.

En effet, dans l'exemple de réalisation illustré sur la figure, ces moyens de maintien comprennent un ou plusieurs organes en forme de peigne de guidage répartis sur la hauteur de l'enceinte, pour maintenir l'instrument en position optimale de désinfection.

Dans l'exemple illustré, deux séries de deux peignes complémentaires désignés respectivement par les références 8, 9, 10 et 11 sont utilisés à deux hauteurs différentes dans l'enceinte pour maintenir l'instrument dans la position optimale.

Il va de soi bien entendu que d'autres modes de réalisation de ces moyens peuvent être envisagés. Ainsi et à titre d'exemple un ou plusieurs tubes de réception d'un ou de plusieurs instruments peuvent également être envisagés, ces tubes s'étendant par exemple au voisinage du centre de l'enceinte dans une position verticale.

Cependant, et selon un autre mode de réalisation encore, ces moyens de maintien peuvent également être formés par un poids adapté pour être accroché et/ou suspendu à l'extrémité libre correspondante de l'instrument afin de maintenir celui-ci en position.

Bien entendu et comme cela a été indiqué précédemment, ces moyens de maintien sont réalisés en matériau transparent au rayonnement tel que par exemple à un rayonnement UV de type C.

Dans ce cas, ces moyens de maintien sont réalisés à titre d'exemple en oxyde de silicium.

Il va de soi bien entendu que d'autres modes de réalisation peuvent encore être envisagés.

Ce maintien en position de l'instrument permet alors d'assurer qu'il est dans la position la plus favorable possible à sa désinfection dans l'enceinte.

## Revendications

1. Enceinte de désinfection d'instruments médicaux comprenant une partie active et une partie de raccordement sous la forme d'un câble, du type comportant des moyens (2) de suspension d'au moins un instrument (4) dans l'enceinte, et des moyens de génération dans l'enceinte d'un rayonnement de désinfection, et des moyens (7), transparents au rayonnement, de maintien de l'instrument (4) dans un état allongé et une orientation sensiblement verticale, sous les moyens de suspension (2), **caractérisé en ce que** les moyens de maintien comprennent des moyens de guidage (8, 9, 10, 11) de l'instrument dans l'enceinte formés de deux séries de deux organes en forme de peigne réparties sur la hauteur de l'enceinte (1).

2. Enceinte de désinfection d'instruments médicaux selon la revendication 1, **caractérisée en ce que** les moyens de maintien sont adaptés pour maintenir l'instrument (4) sensiblement au centre de l'enceinte (1).

3. Enceinte de désinfection d'instruments médicaux selon la revendication 3, **caractérisée en ce que** les moyens de guidage comprennent un ou plusieurs tubes de réception de l'instrument (4).

4. Enceinte de désinfection d'instruments médicaux selon l'une quelconque des revendications précédentes, **caractérisée en ce que** les moyens de maintien comprennent un poids adapté pour être accroché à l'extrémité libre de l'instrument (4).

5. Enceinte de désinfection d'instruments médicaux selon l'une quelconque des revendications précédentes, **caractérisée en ce que** les moyens de maintien sont réalisés en oxyde de silicium.

## Claims

1. Disinfection chamber for medical instruments comprising an active part and a connection part in the form of a cable, of the type having devices (2) for suspending at least one instrument (4) in the chamber and devices for generating a radiation for disinfection in the chamber and devices (7) that are transparent to the radiation for holding the instrument (4) in an extended state and oriented substantially vertically under the suspension devices (2), **characterised in that** the holding devices comprise devices (8, 9, 10, 11) for guiding the instrument in the chamber formed by two series of two comb-like members distributed over the height of the chamber (1).

2. Disinfection chamber for medical instruments according to claim 1, **characterised in that** the holding devices are arranged to hold the instrument (4) substantially in the centre of the chamber (1).

3. Disinfection chamber for medical instruments according to claim 3, **characterised in that** the guide devices comprise one or more receiving tubes for the instrument (4).

4. Disinfection chamber for medical instruments according to any one of the preceding claims, **characterised in that** the holding devices comprise a weight arranged to be hooked onto the free end of the instrument (4).

5. Disinfection chamber for medical instruments according to any one of the preceding claims, **characterised in that** the holding devices are made from silicon oxide.

## Patentansprüche

1. Desinfektionskammer für medizinische Instrumente mit einem aktiven Teil und einem Verbindungsteil in der Form eines Kabels, von dem Typ, der Mittel (2) zur Aufhängung zumindest eines Instruments (4) in der Kammer und Mittel zur Erzeugung einer Desinfektionsstrahlung innerhalb der Kammer und für Strahlung transparente Mittel (7) zum Halten des Instruments (4) in einem ausgestreckten Zustand und in einer im Wesentlichen vertikalen Orientierung unter den Aufhängungsmitteln (2) umfasst,
**dadurch gekennzeichnet, dass** die Haltemittel Führungsmittel (8, 9, 10, 11) für das Instrument in der Kammer umfassen, die aus zwei Reihen aus zwei kammförmigen Organen gebildet sind, die über die Höhe der Kammer (1) verteilt sind.

2. Desinfektionskammer für medizinische Instrumente nach Anspruch 1, **dadurch gekennzeichnet, dass** die Haltemittel ausgebildet sind, um das Instrument (4) im Wesentlichen in der Mitte der Kammer (1) zu halten.

3. Desinfektionskammer für medizinische Instrumente nach Anspruch 3, **dadurch gekennzeichnet, dass** die Führungsmittel eine oder mehrere Aufnahmeröhren für das Instrument (4) umfassen.

4. Desinfektionskammer für medizinische Instrumente nach einem der vorhergehenden Ansprüche, **dadurch gekennzeichnet, dass** die Haltemittel ein Gewicht umfassen, das ausgebildet ist, um an dem freien Ende des Instruments (4) eingehängt zu werden.

5. Desinfektionskammer für medizinische Instrumente nach einem der vorhergehenden Ansprüche, **dadurch gekennzeichnet, dass** die Haltemittel aus Siliziumoxid realisiert sind.
